Europäisches Patentamt

**European Patent Office**

Office européen des brevets

(19)

(11) Numéro de publication: **0 033 017**
**B1**

(12)

# FASCICULE DE BREVET EUROPEEN

(45) Date de publication du fascicule du brevet: **04.01.84**

(51) Int. Cl.³: **C 12 M 1/00**

(21) Numéro de dépôt: **80108259.5**

(22) Date de dépôt: **31.12.80**

(54) **Procédé et dispositif pour la production de biométhane par fermentation anaérobie de pailles, fumiers et matériaux analogues.**

(30) Priorité: **15.01.80 FR 8000803**

(43) Date de publication de la demande:
**05.08.81 Bulletin 81/31**

(45) Mention de la délivrance du brevet:
**04.01.84 Bulletin 84/1**

(84) Etats contractants désignés:
**AT BE CH DE GB IT LI NL SE**

(56) Documents cités:
**FR - A - 1 082 647**
**GB - A - 2 013 170**

(73) Titulaire: **SOCIETE NATIONALE ELF AQUITAINE**
**Tour Aquitaine**
**F-92400 Courbevoie (FR)**

(72) Inventeur: **Goma, Gérard**
**Avenue de Rangueil**
**F-31077 Toulouse (FR)**

(74) Mandataire: **Hirsch, Marc-Roger et al,**
**34 rue de Bassano**
**F-75008 Paris (FR)**

Courier Press, Leamington Spa, England.

Procede et dispositif pour la production de biomethane par fermentation
anaerobie de pailles, fumiers et materiaux analogues

La présente invention se rapporte à un procédé et à un dispositif pour la production de gaz dit gaz de fumier ou biométhane par la fermentation anaérobie de pailles, fumiers et autres déchets de mêmes nature.

Il est connu de produire un tel gaz par fermentation de déchets d'élevage et de pailles dans des fermenteurs ou digesteurs, cette fermentation méthanique s'effectuant à l'abri de l'air sous l'action de microorganismes tels les bactéries du rumen, ou celles rencontrées dans les digesteurs.

Ce biométhane est utilisable comme source d'énergie, et apparaît notamment comme particulièrement approprié ou fonctionnement des installations agricoles.

Les boues qui résultent de la digestion du fumier sont particulièrement riches en azote et constituent à ce titre un excellent engrais.

L'invention a pour objet la réalisation d'un dispositif pour la fermentation de fumiers, naturels ou non, et autres déchets du même genre, en vue d'obtenir un gaz, ou biogaz, plus précisément un gaz contenant du méthane, utilisable comme source d'énergie, pour le chauffage ou pour des moteurs à combustion.

Elle a aussi pour objet un procédé mis en oeuvre dans ce dispositif, qui permet notamment de réduire les risques d'inhibition de la réaction de fermentation, d'améliorer la concentration et l'efficacité de la population active qui intervient dans la fermentation et qui permet, par recyclage, de créer des processus de sélection conduisant à une augmentation de la résistance des microorganismes constituant cette population active.

Le dispositif selon l'invention pour la fermentation anaérobie de fumier en vue de la production de biométhane comporte un réacteur à la partie basse duquel est introduit le fumier dans un milieu aqueux réactionnel, ce fumier progressant pendant la fermentation vers le haut du réacteur où s'effectue le prélèvement de gaz produit séparé des solides, ce réacteur comportant une cloison verticale perforée le séparant intérieurement en deux compartiments, le premier recevant le fumier et le second, occupé au moins en partie par un garnissage poreux de fixation des microorganismes, assurant la fermentation, et comporte un décanteur de recyclage associé au réacteur, ce décanteur étant alimenté à partir du second compartiment du réacteur par la fraction liquide évacuée du réacteur qu'il sépare en une partie enrichie en microorganismes, recyclée vers ledit second compartiment, et une partie appauvrie qui est rejetée.

On obtient ainsi une grande régularité de fermentation, grâce au maintien de la population active des microorganismes.

D'autres buts et avantages de la présente invention apparaîtront à la lecture de la description suivant et des figures jointes, données, à titre illustratif mais non limitatif.

La Figure 1 illustre schématiquement une forme de réalisation du dispositif selon l'invention.

La Figure 2 est une vue analogue d'une variante de réalisation de ce dispositif.

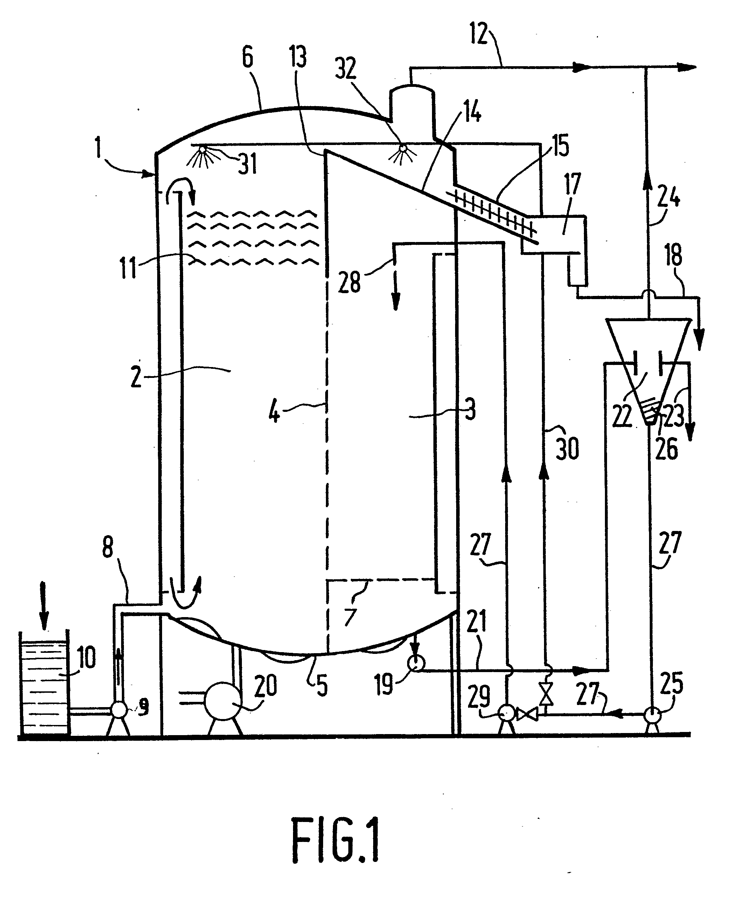

Le dispositif selon l'invention, dans la forme de réalisation de la Figure 1, comprend un réacteur 1, ayant la forme d'un cylindre d'axe vertical, séparé en deux compartiments 2 et 3 par une cloison verticale 4 partant du fond 5 dudit réacteur et s'étendant jusqu'au voisinage de la coupole 6 fermant ce réacteur à sa partie supérieure. Au moins dans sa partie inférieure, la cloison 4 comporte des perforations faisant communiquer les deux compartiments 2 et 3.

A la base du réacteur 1, dans le compartiment 2, aboutit une canalisation de chargement 8, qui, par l'intermédiaire d'une pompe dilacératrice 9, assure l'alimentation du réacteur en pailles, fumiers et autres matières analogues placées dans un réservoir 10 d'ou part ladite canalisation 8.

Les matières 11 hachées par la pompe 9 et introduites dans le compartiment 2, emplissent ce dernier; elles baignent dans le liquide réactionnel de fermentation, chargé en bactéries, et sont surmontées par une atmosphère de gaz résultant de la fermentation, ce gaz qui est un mélange de méthane et de gaz carbonique étant évacué par une canalisation 12 partant du sommet du réacteur, vers un récipient de stockage non représenté.

Le compartiment 3 dans lequel règne la même pression que dans le compartiment 2, est également rempli par le liquide de fermentation, maintenu sensiblement exempt de matières solides grâce à l'effet de filtre de la cloison 4. Un plancher perforé 7 est disposé à la partie inférieure du compartiment 3 et participe à cet effet de filtre. Ce compartiment 3 comporte intérieurement un garnissage poreux permettant la fixation des microorganismes qui assurent la fermentation.

Bien entendu, le niveau des matières monte dans le compartiment 2, au fur et à mesure de l'introduction des nouvelles matières et, à sa partie supérieure 13, la cloison forme trop-plein et se prolonge par un plan incliné 14 conduisant à un extracteur 15 à vis sans fin communiquant, par l'intermédiaire d'un sas étanche 17 avec une canalisation d'évacuation 18 des solides. L'objet du sas 17 est d'éviter toute entrée d'air et d'empêcher un mélange de cet air avec le méthane produit.

A la partie inférieure du compartiment 2 est prévu un dispositif de débourbage 20.

En même temps qu'on extrait du compartiment 2 les matières solides en excès, on soutire du compartiment 3 le liquide égalemene en

excés pour tenir compte notamment de l'apport de matière.

Ce soutirage est effectué au moyen d'une pompe 19 et par une canalisation 21, à la partie inférieure du compartiment 3 (Figure 1), cette canalisation aboutissant à un décanteur 22. Dans ce décanteur 22, on opère une séparation de la phase gazeuse dirigée par un conduit 24 vers la canalisation 12, et la séparation de la phase liquide en une fraction appauvrie en microorganismes évacuée en 23 et en une fraction enrichie en ces microorganismes, contenant par exemple de 5 à 100 g de microorganismes par litre, reprise dans le fond 26 du décanteur par une canalisation de recyclage 27 et une pompe 25.

Le liquide ainsi enrichi en bactéries est introduit en 28 par la canalisation 27, équipée d'une pompe 29, à la partie supérieure du compartiment 3, les bactéries introduites se fixant dans le garnissage de ce compartiment.

De la canalisation 27 part une dérivation 30 débouchant dans le réacteur à la partie supérieure de celui-ci, et se terminant par des orifices d'arrosage 31 et 32, au-dessus du compartiment 2 et du plan incliné 14. Cet apport de liquide facilite la sortie des solides et, part humidification de ceux-ci, facilite aussi la descente des microorganismes dans le compartiment 2.

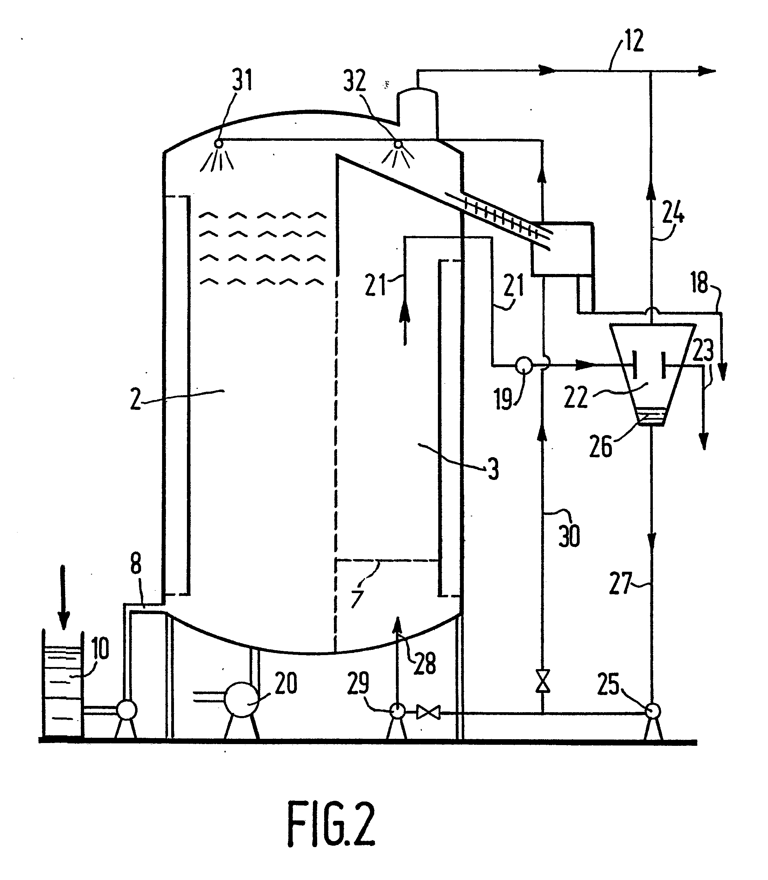

La forme de réalisation de la Figure 2 ne diffère de celle qui vient d'être décrite que par la disposition relative des éléments du circuit de recyclage, lesquels, par ailleurs, ont reçu les mêmes références.

Ainsi, le soutirage de liquide s'opère par la canalisation 21, à la partie supérieure du compartiment 3, et la séparation étant effectuée dans le décanteur 22, la fraction liquide enrichie en microorganismes est introduite en 28, dans ce même compartiment 3, à la partie inférieure de celui-ci.

Les autres éléments du dispositifs sont inchanges.

A titre seulement indicatif, on peut noter que le temps de séjour des matières dans le réacteur, nécessaire pour fermentation complète, va de 4 à 30 jours.

Un réacteur de 2 m³—la densité des matières sèches étant d'environ 0,15—contient environ 300 kg de matières. Un tel réacteur consomme, par jour, de 10 à 75 kg de matières sèches, selon les cas, et 1 kg de fumier donne par fermentation de 0,45 à 0,5 m³ de biogaz, il produit environ de 3 à 20 m³ d'un mélange de $CO_2$ et de $CH_4$, dans un rapport de 40/60, par m³ de réacteur et par jour.

Bien entendu, la présente invention n'est nullement limitée aux modes de réalisation décrits et représentés.

**Revendications**

1. Dispositif pour la fermentation anaérobie de fumier en vue de la production de biométhane, ce dispositif étant caractérisé en ce qu'il comporte un réacteur à la partie basse duquel est introduit le fumier dans un milieu aqueux réactionnel, ce fumier progressant pendant la fermentation vers le haut du réacteur où s'effectue le prélèvement de gaz produit séparé des solides, en ce que ce réacteur comporte une cloison verticale perforée le séparant intérieurement en deux compartiments, le premier recevant le fumier et le second, au moins en partie, occupé par un garnissage poreux de fixation des microorganismes, assurant la fermentation, et en ce qu'un décanteur de recyclage est associé au réacteur, ce décanteur étant alimenté à partir du second compartiment du réacteur par la fraction liquide évacuée du réacteur qu'il sépare en une partie enrichie en microorganismes recyclée vers ledit second compartiment, et une partie appauvrie qui est rejetée.

2. Dispositif selon la revendication 1, caractérisé en ce que le décanteur est alimenté par une canalisation partant de la partie basse du réacteur, le recyclage du liquide enrichi en microorganismes s'effectuant en partie haute dudit réacteur.

3. Dispositif selon la revendication 1, caractérisé en ce que le décanteur est alimenté par une canalisation partant de la partie haute du réacteur, le récyclage du liquide enrichi en microorganismes s'effectuant en partie basse dudit réacteur.

4. Dispositif selon l'une quelconque des revendications 1 à 3, caractérisé par des moyens d'extraction des solides à la partie supérieure du premier compartiment, communiquant avec l'extérieure par un sas étanche.

5. Dispositif selon la revendication 4, caractérisé en ce que les moyens d'extraction des solides sont constitués par une vis sans fin.

6. Dispositif selon l'une quelconque des revendications 1 à 5, caractérisé en ce que des moyens d'arrosage et d'humidification disposés à la partie supérieure du reacteur, à l'intérieure de celui-ci, sont reliés à une dérivation partant de la canalisation de recyclage du décanteur.

7. Dispositif selon l'une quelconque des revendications 1 à 6, caractérisé en ce que le décanteur comporte une canalisation de départ de gaz reliée au prélèvement de gaz du réacteur.

8. Dispositif selon l'une quelconque des revendications 1 à 7, caractérisé en ce qu'un dispositif de débourbage communique avec la partie inférieure du premier compartiment du réacteur.

9. Procédé de fermentation, mis en oeuvre dans le dispositif selon l'une quelconque des revendications 1 à 8, caractérisé en ce qu'on fait décanter le liquide contenant les microorganismes qui assurent la fermentation, et en ce qu'on ramène la fraction enrichie en ces microorganismes dans le second compartiment du réacteur pour que lesdites microorganismes se fixent sur le garnissage de ce compartiment.

## Patentansprüche

1. Vorrichtung zur anaeroben Vergärung von Mist zwecks Herstellung von Biomethangas, welche Vorrichtung dadurch gekennzeichnet ist, dass sie ein Reaktionsgefäss aufweist, in dessen unteren Teil der Mist in ein wässeriges Reaktionsmedium eingegeben wird, wobei der Mist während der Gärund zum oberen Teil des Reaktionsgefässes hin aufsteigt, wo das hergestellte, von den Feststoffen getrennte Gas abgezogen wird, dass eine senkrechte, perforierte Trennwandung des Reaktionsgefässes den Innenraum des letzteren in zwei Kammern teilt, deren erste den Mist aufnimmt, während die zweite Kammer wenigstens teilweise mit einer porösen, die die Gärung hervorrufenden Mikroorganismen fixierenden Garnitur gefüllt ist, und dass mit dem Reaktionsgefäss ein Umlaufabscheider verbunden ist, der von der zweiten Kammer des Reaktionsgefässes mit einer flüssigen Fraktion beschikt wird, die er in einem zur zweiten Kammer zurückgeführten, mit Mikroorganismen angereicherten Anteil und einen verarmten Anteil aufteilt, welch letzterer entfernt wird.

2. Vorrichtung nach Anspruch 1, dadurch gekennzeichnet, dass der Abscheider von einer an den unteren Teil des Reaktionsgefässes angeschlossenen Leitung gespeist wird, während die mit Mikroorganismen angereicherte Flüssigkeit in den oberen Teil des Reaktionsgefässes zurückgeführt wird.

3. Vorrichtung nach Anspruch 1, dadurch gekennzeichnet, dass der Abscheider von einer an den oberen Teil des Reaktionsgefässes angeschlossenen Leitung gespeist wird, während die mit Mikroorganismen angereicherte Flüssigkeit in den unteren Teil des Reaktionsgefässes zurückgeführt wird.

4. Vorrichtung nach einem der Ansprüche 1 bis 3, dadurch gekennzeichnet, dass im oberen Teil der ersten Kammer über eine dichte Schleuse mit dem Ausseren verbundene Mittel zum Abziehen der Feststoffe vorgesehen sind.

5. Vorrichtung nach Anspruch 4, dadurch gekennzeichnet, dass die Feststoffabziehmittel eine Schnecke umfassen.

6. Vorrichtung nach einem der Ansprüche 1 bis 5, dadurch gekennzeichnet, dass innerhalb des oberen Teiles des Reaktionsgefässes Besprengungsund Befeuchtungsmittel angeordnet und mit einer an die Rückführungsleitung des Abscheiders angeschlossen Abzweigleitung verbunden sind.

7. Vorrichtung nach einem der Ansprüche 1 bis 7, dadurch gekennzeichnet, dass sie eine mit dem unteren Teil der ersten Kammer des Reaktionsgefässes verbundene Ausschlämmeinrichtung aufweist.

8. Vorrichtung nach einem der Ansprüche 1 bis 7, dadurch gekennzeichnet, dass sie eine mit dem unteren Teil der ersten Kammer des Reaktionsgefässes verbundene Ausschlämmeinrichtung aufweist.

9. Vermittels der Vorrichtung nach einem der Ansprüche 1 bis 8 durchgeführtes Gärverfahren, dadurch gekennzeichnet, dass die die Gärung bewirkenden Mikroorganismen enthaltende Flüssigkeit dekantiert wird, und dass die mit diesen Mikroorganismen angereicherte Fraktion in die zweite Kammer des Reaktionsgefässes zurückgeführt wird, so dass die genannten Mikroorganismen auf der Garnitur dieser Kammer fixiert werden.

## Claims

1. Device for the anaerobic fermentation of manure with a view to the production of biomethane, this device being characterized in that it comprises a reactor, into the bottom part of which is introduced the manure in a reactional aqueous medium, this manure progressing during fermentation towards the top part of the reactor where the drawing off of the gas produced, separated from the solids is carried out, in that this reactor comprises a perforated vertical partition separating it internally into two compartments, the first receiving the manure and the second, at least in part, occupied by a porous packing of fixation of the microorganisms, ensuring the fermentation, and in that a recycling decanter is associated to the reactor, this decanter being fed from the second compartment of the reactor by the liquid fraction evacuated from the reactor that it separates into one part enriched in recycled microorganisms towards the second compartment, and a second impoverished part that is rejected.

2. Device according to claim 1, characterized in that the decanter is fed by a pipe issuing from the bottom part of the reactor, the recycling of the part enriched in microorganisms being carried out in the top part of said reactor.

3. Device according to claim 1, characterized in that the decanter is fed by a pipe issuing from the top part of the reactor, the recycling of the liquid enriched in microorganisms being carried out at the bottom part of the said reactor.

4. Device according to any one of claims 1 to 3, characterized in that the means for extracting the solids at the top part of the first compartment communicate with the outside by a sealed lock chamber.

5. Device according to claim 4, characterized in that the means for extracting the solids are constituted by a worm screw.

6. Device according to any one of claims 1 to 5, characterized in that the spraying and humidification means disposed at the top part of the reactor, inside said reactor, are connected to a derivation issuing from the recycling pipe of the decanter.

7. Device according to any one of claims 1 to 6, characterized in that the decanter comprises a gas issue pipe connected to the drawing off of the gas of the reactor.

8. Device according to any one of claims 1 to 7, characterized in that a clearing device communicates with the bottom part of the first compartment of the reactor.

9. Fermentation process, operated in the device according to any one of claims 1 to 8, characterized in that the liquid containing the microorganisms that ensure the fermentation is made to decant, and in that the fraction enriched in these microorganisms is brought into the second compartment of the reactor so that the said microorganisms are fixed on the packing of this compartment.

FIG.1

1

FIG.2